# EUROPÄISCHE PATENTSCHRIFT

(11) **EP 1 128 775 B1**
(45) Veröffentlichungstag und Bekanntmachung des Hinweises auf die Patenterteilung: **28.06.2006**
(21) Anmeldenummer: 99941591.2
(22) Anmeldetag: 11.08.1999
(51) Int. Cl.: A61B 18/20

(54) **DERMATOLOGISCHES HANDSTÜCK**
DERMATOLOGICAL HAND PIECE
PIECE A MAIN DERMATOLOGIQUE

(30) Priorität: 12.11.1998 DE 19852948
(43) Veröffentlichungstag der Anmeldung: 05.09.2001
(73) Patentinhaber: Carl Zeiss Meditec AG, 07745 Jena (DE)
(72) Erfinder: ELBRECHT, Jens, D-07751 Jena (DE); KÜHNERT, Jürgen, D-07749 Jena (DE); SEITZ, Bernhard, D-07751 Jenapriessnitz (DE); ZIMMERMANN, Gabriele, D-07747 Jena (DE)
(74) Vertreter: Geyer, Fehners & Partner
(86) Internationale Anmeldenummer: PCT/EP1999/005888
(87) Internationale Veröffentlichungsnummer: WO 2000/028910

(56) Entgegenhaltungen:
- EP-A- 0 783 904
- EP-A- 0 827 716
- WO-A-91/04829
- WO-A-95/18984
- WO-A-98/51235
- WO-A-98/52481
- DE-A- 19 623 749
- FR-A- 2 738 082
- US-A- 5 658 275
- US-A- 5 738 681
- US-A- 5 830 208

## Beschreibung

### Gebiet der Erfindung

Die Erfindung bezieht sich auf ein Handstück zur kosmetischen Behandlung von Hautflächen, von dem ein Laserstrahl auf eine ausgewählte Hautstelle gerichtet ist und diese der Einwirkung des Laserstrahles unterzogen wird.

### Stand der Technik

Derzeit werden Laser weltweit zur kosmetischen Behandlung der Haut, vorwiegend von vaskulären und pigmentierten Läsionen, wie z.B. zur Entfernung von Feuermalen und von Tätowierungen, zur Hautemeuerung und auch zur Haarentfernung genutzt. Dabei werden meist kurze Laserimpulse mit einer Impulsdauer vom Nano- bis zum Millisekundenbereich in das Gewebe eingebracht. Derartige Behandlungen dienen vorwiegend der Verbesserung der Lebensqualität der behandelten Personen und sind in der Regel der Kosmetik zuzuordnen.

Die Geräte zur Durchführung solcher Behandlungen umfassen im wesentlichen eine Laserstrahlungsquelle und ein Handstück, das zur Ausrichtung der von der Laserstrahlungsquelle emittierten Strahlung auf die Hautstelle dient, die behandelt werden soll.

Um eine leichtgewichtige Bauweise des Handstückes zu erzielen und so eine möglichst ungehinderte Manipulation mit dem Handstück ermöglichen zu können, sind Laserstrahlungsquelle und Handstück als getrennte Baugruppen ausaeführt, wobei die Übertragung der Laserstrahlung von der Strahlungsquelle zum Handstück mittels einer beweglichen Strahlführungseinrichtung erfolgt. Die Strahlführungseinheit kann aus mehreren, durch Gelenke miteinander verbundenen starren Übertragungsgliedern bestehen, als biegsame Faseroptik oder aus anderweitig ausgebildet sein.

Das Handstück weist am Übergang von der Strahlführungseinheit ein Einkoppelelement auf, und an dem auf die Haut zu richtende Ende des Handstückes ist eine Abstrahlfläche für die Laserstrahlung vorhanden. Oftmals ist ein Abstandhalter vorgesehen, der die Arbeitsebene festlegt und somit sicherstellt, daß Querschnitt und Intensität des applizierten Laserstrahles in der Arbeitsebene den gewählten Parametern entsprechen.

Für viele dermatologische Anwendungen ist es vorteilhaft, die äußeren Hautschichten zur Vermeidung von Schädigungen durch die Laserstrahlung zu kühlen. Hierzu sind verschiedene Verfahren und Vorrichtungen bekannt.

So ist es beispielsweise üblich, Kühl-Gel auf die zu behandelnde Fläche aufzutragen oder die zu behandelnde Fläche mit einem Spray zu kühlen. Hierbei ist es jedoch schwierig, die Temperatur auf der Haut und in den unmittelbar darunter liegenden Schichten so zu beeinflussen, daß einerseits der gewünschte Behandlungseffekt realisiert, andererseits aber eine Schädigung der Epidermis durch die Laserstrahlung weitgehend vermieden wird. Ein weiterer Nachteil besteht darin, daß eine lokale gleichmäßige Kühlung nicht mit Sicherheit erreicht wird.

In der US-Patentschrift 5,057,104 ist ein Verfahren und eine Vorrichtung zur Behandlung "cutaneous vascular lesions" beschrieben, wobei der Laserstrahl durch einen stationären, mit dem zu behandelnden Hautabschnitt in Verbindung stehenden Kühlcontainer geführt wird. Auf diese Weise wird der Hautstelle während der Behandlung Wärme entzogen.

In der US-Patentschrift US 5,735,844 ist eine Vorrichtung zur Haarentfernung dargestellt, bei der eine optisch transparente Linse, durch welche der Laserstrahl auf die zu behandelnde Hautstelle gerichtet ist, sowohl in Kontakt mit einer Kühleinheit als auch mit der Haut gebracht wird. Dabei entzieht die Linse ebenfalls wie oben beschrieben während der Behandlung der betreffenden Hautstelle Wärme.

Als nachteilig bei den vorgenannten Vorrichtungen erweist sich der große Platzbedarf, der durch die Ausdehnung der zur Kühlung auf die Haut aufgesetzten Mittel bedingt ist. Das ist bei der Behandlung kleiner Flächen hinderlich. Weiterhin ist die Wärmeleitfähigkeit der verfügbaren, für die Laserstrahlung transparenten Materialien vergleichsweise gering, so daß eine schnelle und optimale Kühlung nicht erreicht werden kann.

Ein weiterer wesentlicher Nachteil besteht darin, daß bel der Positionierung der Austrittsoptik für den Laserstrahl und der Kühleinrichtung zunächst mit der Einstrahlung der Laserenergie gewartet werden muß, bis die zu behandelnde Hautstelle optimal abgekühlt ist. Das ist besonders dann nachteilig, wenn eine größere Hautfläche zu behandein ist, auf die die Laserstrahlung in mehrere nebeneinanderliegende Hautstellen eingebracht werden muß. Die Behandlungsdauer ist bedingt durch den Umstand, daß für jede dieser Hautstellen erst eine Verweilzeit zur Kühlung erforderlich ist und dann erst die Lasereinstrahlung erfolgen kann, verhältnismäßig groß.

Auch eine Nachbehandlung der betreffenden Hautabschnitte durch Temperierung ist mit den bekannten Handstücken nicht möglich.

Die internationale Anmeldung WO98/51235, die Stand der Technik unter Art.54(3) EPÜ ist, offenbart ein Gerät zur Behandlung dermatologischer Probleme mittels Laserlicht, wobei ferner Mittel zur Temperierung der Haut vorgesehen sind. Die Grösse der Temperier- und Laserapplikationsflächen sind in Abhängigkeit der thermischen Parameter so dimensioniert, dass das Gerät mit konstanter Geschwindigkeit über die zu behandelnde Fläche geführt werden kann.

### Beschreibung der Erfindung

Von diesem Stand der Technik ausgehend liegt der Erfindung die Aufgabe zugrunde, ein Handstück zur kosmetischen Behandlung von Hautstellen mittels Laserstrahlung so weiterzubilden, daß eine effizientere Behandlung bei weirestgehender Schonung der Haut gewährleistet ist.

*Bei einem Handstück, von dem ein Laserstrahl auf die Oberfläche einer ausgewählten Hautfläche gerichtet ist, wobei die Haut der Einwirkung des Laserstrahles unterzogen wird und wobei der Laserstrahl in zeitlicher Folge fortschreitend auf einzeine, dem Laserstrahlquerschnitt entsprechende, in ihrer Gesamtheit die ausgewählte Hautfläche überdeckende Hautstellen gerichtet wird, und das ausgestattet ist mit einer Einrichtung zum Temperieren der Hautfläche, bei der mindestens eine temperierte Kontaktfläche seitlich neben dem auf eine Hautstelle gerichteten Laserstrahl positioniert ist, ist erfindungsgemäß vorgesehen, daß*
- *die Form der Kontaktfläche der Form des Laserstrahlquerschnittes entspricht,*
- *die flächige Ausdehnung der Kontaktfläche dem 0,5- bis 2-fachen der flächigen Ausdehnung der Querschnittsfläche des Laserstrahles entspricht, und*
- *der Abstand zwischen Laserstrahl und Kontaktfläche so bemessen ist, daß in zeitlich aufeinannder folgenden Schritten der Laserstrahl und die neben dem Laserstrahl positionierte Kontaktfläche so auf die Hautstellen gerichtet sind, daß*
- *jeweils während einer vorgegebenen Einwirkzeit*
   - *der Laserstrahl ortsunveränderlich auf eine Hautstelle gerichtet ist und dabei zugleich die Kontaktfläche*
   - *mit einer zuvor der Laserstrahlung ausgesetzten Haurstelle oder*
   - *mit einer nachfolgend der Laserstrahlung auszusetzende Hautstelle in Berührung steht.*

Der Abstand zwischen Laserstrahl und einer solchen Kontaktfläche ist erfindungsgemäß so bemessen, daß gleichzeitig mit der Ausrichtung des Laserstrahles auf eine zweite Hautstelle eine erste Hautstelle, auf die der Laserstrahl vorher gerichtet war und auf die der Laserstrahl soeben eingewirkt hat, mit der Kontaktfläche in Berührung steht. Alternativ dazu oder auch zusätzlich kann eine weitere Kontaktfläche vorhanden sein, deren Abstand zum Laserstrahl so bemessen ist, daß diese mit einer dritten Hautstelle in Berührung steht, auf die der Laserstrahl im nächsten Schritt einwirken soll (während gleichzeitig der Laserstrahl noch auf die zweite Hautstelle gerichtet ist und die erste Hautstelle noch mit der ersten Kontaktfläche in Berührung steht).

Sinngemäß können am Handstück weitere Kontaktstellen vorgesehen sein, die während der Ausrichtung und Einwirkung des Laserstrahles auf die zweite Hautstelle mit weiteren vorher behandelten Hautstellen und/oder mit weiteren nachfolgend zu behandelnden Hautstellen zwecks Temperierung in Berührung stehen.

Mit anderen Worten: die Kontaktflächen sind in bezug auf die Laserstrahlung am Handstück so positioniert, daß während der Einwirkung des Laserstrahles auf eine Hautstelle jeweils mindestens eine weitere Hautstelle, auf die der Laserstrahl vorher (vor Umsetzung des Handstückes) eingewirkt hat und/oder auf die der Laserstrahl nachfolgend (nach Umsetzung des Handstückes) einwirken soll, mit einer Kontaktfläche in Berührung stehen. Auf diese Weise wird erreicht, daß die einzelnen Hautstellen in unmittelbarer Vorbereitung auf die Laserstrahleinwirkung und/oder zwecks Nachbehandlung temperiert werden. Das Handstück kann daher ohne Verzögerung von Hautstelle zu Hautstelle fortschreitend umgesetzt werden.

In Ausgestaltungsvarianten kann die Ausdehnung der Kontaktfläche kleiner oder größer ausgeführt sein als die Ausdehnung des Laserstrahlquerschnittes. Damit ist es beispielsweise möglich, einen größeren Flächenabschnitt vor der Behandlung zu temperieren, wodurch mit erhöhter Sicherheit nur vortemperierte Flächenabschnitte der Einwirkung des Laserstrahles unterzogen werden. Die Temperierung einer im Vergleich zum Laserstrahlquerschnitt kleineren Fläche kann der schonenderen Behandlung bestimmter Hautstellen dienen.

Erfindungsgemäß ist die mindestens eine Kontaktfläche mit einem Kühl- und/oder Heizaggregat wärmeleitend verbunden. Vorzugsweise ist ein Kühlaggregat vorgesehen. Dieses kann beispielsweise als Peltierelement ausgebildet sein, dessen kalte Seite wärmeleitend mit der Kontaktfläche in Verbindung steht und von dessen warmer Seite über ein in einem Kühlkreislauf umlaufendes Medium die der Hautstelle entzogene Wärme abtransportiert wird.

Denkbar ist alternativ hierzu auch, daß die Kontaktfläche während ihres Aufsitzens auf der vorzutemperierenden Hautstelle wärmeleitend mit einem sich entspannenden und dabei abkühlenden Gas, beispielsweise Stickstoff oder Kohlendioxid, in Verbindung gebracht wird.

In einer besonders bevorzugten Ausgestaltung ist am Handstück ein Temperatursensor vorgesehen, der mit der Kontaktfläche und/oder mit der zur Behandlung ausgewählten und zu temperierenden Hautstelle in Verbindung steht. Mit Hilfe dieses Temperatursensors wird ermittelt, ob die Hautstelle die zur Behandlung erforderliche und für eine erfolgreiche Behandlung vorauszusetzende Temperatur erreicht hat. Das Ausgangssignal des Temperatursensors kann als Einschalt- oder Steuersignal für das Kühl- und/oder Heizaggregat genutzt werden. Auf diese Weise ist es möglich, je nach Bedarf die Temperatur der ausgewählten Hautstelle weiter anzuheben oder abzusenken.

In einer weiteren Ausgestaltung ist vorgesehen, daß der Laserstrahlquerschnitt von einer ringförmigen Fläche umgeben ist, die während der Behandlung auf der ausgewählten Hautstelle aufsitzt. Mit dieser ringförmigen Fläche kann die Hautstelle einem Druck ausgesetzt werden, der sich günstig auf den Behandlungserfolg auswirkt, denn durch die Flächenpressung zwischen der ringförmigen Fläche und der Haut reduziert sich die Dicke der Epidermis, wodurch ein effektiveres Eindringen der Laserenergie in die Haut erzielt wird.

Eine zusätzliche Ausgestaltung sieht vor, daß das Handstück anstelle der ringförmigen Fläche mit Gleitschienen oder Führungsrollen versehen ist, die eine manuelle Geradführung über die Hautfläche hinweg erleichtern. Mit derartigen Gleitschienen oder Führungsrollen ist zugleich auch stets der erforderliche Abstand der Abstrahlfläche zur Haut gewährleistet.

Vorteilhaft können die Gleitschienen als Filterglasscheiben ausgebildet sein, durch die zugleich ein Schutz des Operateurs vor der Laserstrahlung gegeben ist.

Das erfindungsgemäße Handstück kann weiterhin derart ausgestaltet sein, daß innerhalb des Handstückes, der Austrittsfläche der Strahlführungseinrichtung nachgeordnet, mindestens ein optisches Element mit einer im Mikrometerbereich strukturierten und dadurch mikrooptisch wirksamen Fläche vorhanden ist.

Diese Fläche kann eine diffraktiv wirksame Struktur aufweisen, deren Strukturbreite in der Größenordnung der Wellenlänge der zur Behandlung genutzten Laserstrahlung liegt. Eine solche Struktur ist beispielsweise ein variierendes Höhenprofil mit streifenförmigen, kreuzförmigen, trichterförmigen und/oder anderweitig geformten Erhebungen, ein in der genannten Strukturbreite variierter Brechungsindex und/oder variierender Absorptionskoeffizienten. Elemente, die mit derartigen Flächen ausgestattet sind, sind beispielsweise in der Fachliteratur Naumann/Schröder "Bauelemente der Optik", Carl Hanser Verlag München Wien, 6. Auflage, Seite 584 beschrieben.

Mit dieser mikrostrukturierten Fläche wird erreicht, daß sich bei Durchgang der Laserstrahlung durch diese Fläche die Energieverteilung innerhalb des Strahlungsquerschnittes bis in die Randbereiche hinein vergleichmäßigt, d.h. über den gesamten Strahlungsquerschnitt hinweg ist im Strahlengang nach dieser Fläche eine über den Querschnitt vergleichmäßigte Strahlungsintensität vorhanden.

In einer alternativen Ausgestaltung ist auf der Fläche anstelle der diffraktiven eine refraktive wirkende Struktur vorgesehen, beispielsweise aus sphärischen, asphärischen, zylindrischen und/oder elliptischen Linsen, wobei jede der Linsen eine Ausdehnung senkrecht zur Strahlungsrichtung von 10µm bis 1000µm hat. Diese Linsen können auf der Fläche als Array in hexagonalen und/oder orthogonalen Richtungen nebeneinander angeordnet sein. Sie können sowohl als Zerstreuungslinsen konkav oder auch als Sammellinsen konvex geformt sein; es können sowohl konkav als auch konvex geformte Linsen nebeneinander auf der Fläche vorhanden sein. Auch statistisch verteilte konkave Aussparungen, kreisförmig angeordnete bzw. spiralförmig verlaufende Einkerbungen oder auch sich kreuzende Gitter sind denkbar.

Bevorzugte Abmessungen für die refraktiven Strukturen sind Durchmesser von 0,35mm und Tiefen von 0,005mm. Das Verhältnis von Tiefe zu Durchmesser sollte den Wert 0,5 nicht überschreiten. Bei Linsenstrukturen sollte dieser Verhältniswert größer als 0,02 sein und in besonders bevorzugten Ausgestaltungen im Bereich von 0,1 bis 0,3 liegen.

Passiert der Laserstrahl diese Fläche, erfolgt durch die mikrooptisch wirksamen Strukturelemente (Linsen oder Höhenprofile) eine Aufteilung der Strahlung in eine Vielzahl von Teilstrahlen, deren Anzahl von der Anzahl der auf der Fläche vorhandenen Strukturelemente abhängig ist. Je feiner die mikrooptisch wirksame Struktur ausgebildet ist, um so gleichmäßiger und homogener ist die Strahlungsintensität über den gesamten Querschnitt der Laserstrahlung nach Durchgang durch die beschriebene Fläche verteilt. Mit anderen Worten: beim Passieren der mikrostrukturierten Fläche erfolgt die Transformation einer ungleichmäßigen Energieverteilung innerhalb des Strahlquerschnittes in eine bis in die Randbereiche des Strahlquerschnittes hinein vergleichmäßigte Energieverteilung.

Diese Vergleichmäßigung ist insbesondere bei Verwendung eines Rubinlasers als Strahlungsquelle notwendig und vorteilhaft, da dessen Strahlung bekanntermaßen eine stark inhomogene Intensitätsverteilung in ihrem Querschnitt aufweist. Dabei kommt noch hinzu, daß die Intensitätsverteilung in der Rubinlaserstrahlung nicht konstant ist, sondern sich von Spot zu Spot ändert, so daß es bei Verwendung des Rubinlasers zur Haarentfernung ohne die erfindungsgemäß vorgeschlagene Einrichtung leicht zu Verbrennungen kommen kann.

Erfindungsgemäß wird mit der mikrostrukturierten Fläche nicht nur die beabsichtigte Vergleichmäßigung der Intensität innerhalb des Strahlquerschnittes erzielt, sondern je nach Ausbildung der einzelnen Strukturelemente kann weiterhin, sofern das beabsichtigt und gewünscht ist, auch die Richtung der einzelnen Teilstrahlen beeinflußt werden. Das heißt, ein aus einer Faser beispielhaft mit kreisrundem Querschnitt austretender Laserstrahl kann durch gezielt vorgegebene Ausgestaltung der einzelnen Strukturelemente in einen Laserstrahl mit quadratisch, rechteckig, sechseckig oder anderweitig geformtem Strahlquerschnitt übergeführt werden.

Werden nämlich quadratisch, rechteckig oder sechseckig geformte Strahlquerschnitte auf die zu behandelnde Hautstelle gerichtet, lassen sich die einzelnen Spots ohne gegenseitige Überlappung und auch unter Vermeidung unbehandelter Fehlstellen nebeneinander setzen. Mit dem Ausschluß von Überlappungen wird ein zu hoher, mit dem Ausschluß von Fehlstellen ein zu niedriger Energieeintrag in die Hautstellen vermieden und so das Behandlungsergebnis bedeutend verbessert.

Die Umformung des Strahlquerschnittes wird erreicht, indem die Strukturelemente auf der mikrostrukturierten Fläche so ausgewählt, geformt und positioniert sind, daß den Teilstrahlen eine Richtung innerhalb des Laserstrahlquerschnittes gegeben wird, die auf eine gewünschte Außenkontur des Querschnitts zielt. Die Teilstrahlen füllen also nicht mehr einen kreisrunden Strahlquerschnitt, sondern beispielsweise einen quadratisch geformten Querschnitt gleichmäßig aus (die Kreisabschnitte sind ausgespart).

Das erfindungsgemäße Handstück zeichnet sich also gegenüber dem Stand der Technik durch eine über ihren gesamten Querschnitt homogenisierte Intensität der Laserstrahlung an der Abstrahlfläche und außerdem durch eine angepaßte Querschnittsform der Strahlung aus.

Die mikrooptisch wirksamen Strukturen sind beispielsweise mit Hilfe von Elektronenstrahllithographie, Photolithographie oder lonenaustauschverfahren leicht herstellbar.

In einer Ausgestaltung der Erfindung ist vorgesehen, daß der mikrooptisch strukturierten Fläche eine Einrichtung zur Strahlfokussierung vor- oder nachgeordnet ist. Mit dieser Einrichtung läßt sich die Größe des Strahlquerschnittes einstellen. Als derartige Einrichtung kann beispielhaft eine Sammellinse vorgesehen sein, die im Strahlengang vor oder nach der strukturierten Fläche positioniert ist.

Bevorzugt kann als Einrichtung zur Strahlfokussierung eine Zoomoptik vorgesehen sein, mit der es in einfacher Weise möglich ist, die Größe des Spots zu beeinflussen. Ist die Zoomoptik mit einer entsprechenden Stellautomatik gekoppelt, kann die Spotgröße unkompliziert während der Behandlung verändert werden.

In einer weiteren Ausgestaltung der Erfindung ist vorgesehen, daß das optische Element mit der mikrooptisch wirksamen Fläche als strahlführender Stab ausgebildet ist, in welchem die Strahlung durch Totalreflexion weitergeleitet wird. Der Stab verfügt über eine Einstrahlfläche und eine Abstrahlfläche für die Laserstrahlung; dabei ist die Einstrahlfläche mit der mikrooptisch wirksamen Struktur versehen. Der Stab kann aus Quarzglas gefertigt sein. Die Größe und Querschnittsform von Einstrahlfläche und Abstrahlfläche können voneinander abweichen. Vorteilhaft jedoch sollte die Einstrahlfläche kreisrund ausgeführt sein, der kreisrunde Querschnitt sollte über wenigstens 90% der Länge des Stabes erhalten bleiben und erst auf dem verbleibenden Längenabschnitt eine Reduzierung und/oder Formänderung des Querschnitts vorgesehen sein.

Aufgrund der Totalreflexionen innerhalb des strahlführenden Stab wird eine weitere "Durchmischung" der nach dem Passieren der strukturierten Fläche vorhandenen Vielzahl der einzelnen Teilstrahlen erreicht und damit eine weitere Vergleichmäßigung der Strahlungsintensität, bezogen auf den Strahlquerschnitt, bewirkt.

Es ist anzumerken, daß als mikrooptische Strukturen, sofern diese wie erfindungsgemäß vorgesehen auf der Einstrahlfläche eines strahlführenden Stabes ausgebildet sind, auch die Strukturen einer aus dem Stand der Technik bekannten Streuscheibe sein können. Da bei den undefinierbaren Strukturen der Streuscheibe jedoch das Licht auch unter ungünstigen Winkel eintritt, hätte das Rückstrahlungen, Energieverluste und damit auch unerwünscht hohe Wärmeentwicklung zur Folge.

Das wird mit den mikrooptisch wirksamen Strukturen vermieden, da diese so ausgebildet sind, daß ungünstige Eintrittswinkel nicht auftreten. Hier werden gemäß der Fresnelschen Gleichungen (Zusammenhang zwischen Polarisation, Reflexion, Absorption) circa 96 % der Laserstrahlung eingekoppelt, wodurch der Energieverlust und damit auch die Wärmeentwicklung auf ein vertretbares Maß beschränkt ist.

Eine zusätzliche Beeinflussung der über den Querschnitt verteilten Strahlungsintensität läßt sich erzielen, wenn die strukturierte Fläche gewölbt, bevorzugt konkav, besonders bevorzugt konvex ausgebildet ist.

Die Abstrahlfläche kann sowohl einen kreisrunden als auch einen vieleckigen, wie beispielsweise quadratischen oder sechseckigen Querschnitt aufweisen.

Im Rahmen der Erfindung liegen weiterhin Ausgestaltungen, bei denen als Laserstrahlungsquelle ein Rubinlaser oder eine in das Handstück integrierte Laserdiode vorgesehen ist.

Des weiteren kann zwischen der Abstrahlfläche und der zu behandelnden Hautfläche eine Schicht aus einem transparenten Gel, beispielsweise einem Ultraschallgel, vorgesehen sein. Hiermit wird die Einstrahlung des Laserstrahls in die zu behandelnde Hautfläche durch Reduzierung der Reflexion und Verminderung der Streuung weiter optimiert. Dies führt auch dazu, daß für das Laserlicht geringere Energiedichten benötigt werden. Der Brechungsindex des Gels soll dem Brechungsindex der Haut angepaßt sein, das Gel sollte mindestens für die Wellenlänge des verwendeten Laserlichtes transparent sein.

*Beim* Betreiben eines der vorhergehenden Beschreibung entsprechenden Handstückes ist vorgesehen, daß zunächst eine erste Kontaktfläche zum Zwecke der Temperierung auf eine zur Behandlung ausgewählte Hautstelle aufgesetzt wird. Nach einer vorgegebenen Verweilzeit dieser ersten Kontaktfläche auf der Hautstelle wird die Position des Handstückes so verändert, daß sich nun nicht mehr die Kontaktfläche, sondern die Abstrahlfläche für die Laserstrahlung über dieser Hautstelle befindet. Die Kontaktfläche steht nun bereits mit einer weiteren zur Behandlung vorgesehenen Hautstelle, die sich in unmittelbarer Nachbarschaft zur ersten Hautstelle befindet, in wärmeleitendern Kontakt. Während der Verweilzeit der Kontaktfläche auf der zweiten Hautstelle wird die Behandlung der ersten Hautstelle mit der Laserstrahlung vorgenommen.

Nach Abschluß dieser Behandlung, nach der auch die Vorkühlung der zweiten Hautstelle abgeschlossen Ist, wird die Position des Handstückes nun so verändert, daß sich jetzt die Austrittsfläche für die laserstrahlung über der zweiten Hautstelle befindet und die Kontaktfläche mit einer dritten Hautstelle in wärmeleltendem Kontakt befindet und diese vortemperiert. Während dieser Verweilzeit wird die zweite Hautstelle der Behandlung mit der Laserstrahlung unterzogen usw.

Die Positionsänderung des Handstückes von einer behandelten Hautstelle zur nächsten erfolgt durch Umsetzen, wobei das Handstück jeweils aufgesetzt, dann die Strahlungsquelle für die auf diese Hautstelle bezogene Behandlungsdauer ein- und danach wieder ausgeschaltet wird. Im letzteren Fall sind die Laserenergie, die Kühltemperatur an der Kontaktfläche zur Vorkühlung, die Temperatur an der Kontaktfläche zur Nachtemperierung (soweit vorhanden) des Handstückes so aufeinander abgestimmt, daß eine optimale Behandlung erfolgt. Zur Behandlung einer größeren Hautfläche können mehrere derartige "Bahnen" nebeneinander gezogen werden.

*Diese Anwendungsweise* kann dahingehend weiter ausgestaltet sein, daß vor Beginn der Behandlung auf die zu behandelnde Hautfläche ein Gel aufgetragen wird, das für die Wellenlänge des verwendeten Laserlichtes transparent ist und dessen Brechungsindex dem Brechungsindex der Haut angepaßt ist. Damit wird eine effektive Eintragung der Laserenergie in die Haut erzielt, da das von der Haut reflektierte Licht auf einen unwesentlichen Anteil reduziert wird. So werden zugleich auch Nebenwirkungen, die sonst durch Verlustwärme entstehen könnten, vermieden.

Bevorzugt wird ein Ultraschallgel verwendet, dessen Brechungsindex zwischen dem Brechungsindex der Abstrahlfläche und dem Brechungsindex der zu behandelnden Hautfläche liegt. Das Ultraschatlgel ist physiologisch unbedenklich und insofern für kosmetische Zwecke geeignet. Es besitzt außerdem eine gute Wärmeleitfähigkeit.

Außerdem wird durch das Gel die Gefahr der Beschädigung der Epidermis weiter verringert. Die Wirksamkeit kann noch weiter gesteigert werden, indem von der behandelnden Hautstelle vor Beginn der Behandlung eventuell vorhandener Haarwuchs entfernt wird.

### Kurze Beschreibung der Zeichnungen

In den zugehörigen Zeichnungen zeigen:
- Fig.1: die Prinzipdarstellung der erfindungsgemäßen Anordnung in einer ersten Ausgestaltungsvariante
- Fig.2: den Laserstrahlquerschnitt innerhalb des Handstückes nach Fig.1 an verschiedenen Positionen
- Fig.3: eine Ansicht A aus Fig.1
- Fig.4: die Prinzipdarstellung der erfindungsgemäßen Anordnung in einer zweiten Ausgestaltungsvariante

- Fig.5: den Laserstrahlquerschnitt innerhalb des Handstückes nach Fig.4 an verschiedenen Positionen
- Fig.6: eine Ansicht B aus Fig.4
- Fig.7: die Aneinanderreihung von Hautstellen, behandelt mit einer Anordnung nach Fig.1
- Fig.8: die Aneinanderreihung von Hautstellen, behandelt mit einer Anordnung nach Fig.4
- Fig.9: verschiedene Möglichkeiten vortemperierter, behandelter und nachtemperierter benachbarter Hautstellen
- Fig.10: Gestaltungsvarianten einer Einstrahlfläche mit mikrooptischer Struktur im Querschnitt
- Fig.11: Gestaltungsvarianten einer Einstrahlfläche mit mikrooptischer Struktur in Draufsicht
- Fig.12: eine mikrooptische Struktur im Querschnitt

### Ausführliche Beschreibung der Zeichnungen

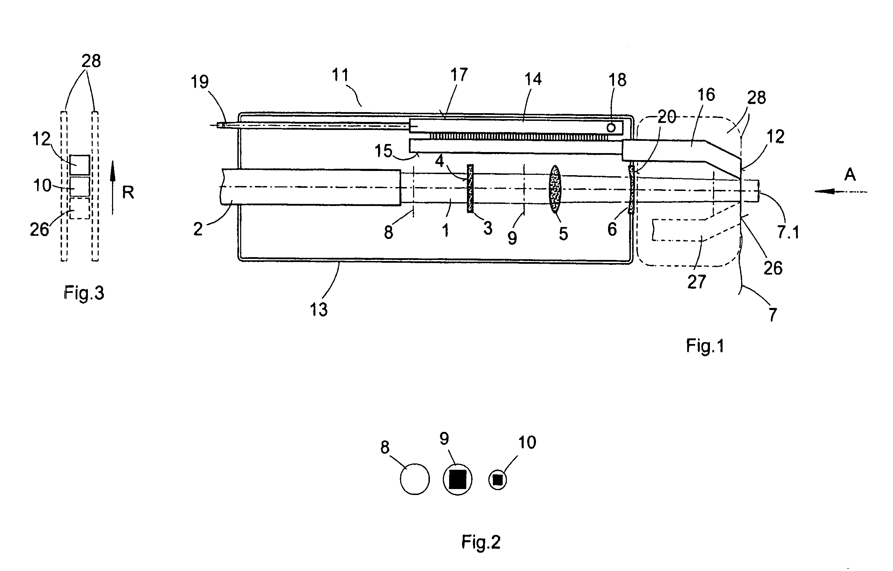

In Fig.1 ist eine erste Ausgestaltungsvariante des erfindungsgemäßen Handstükkes prinzipiell dargestellt. Im Strahlengang 1 einer Laserstrahlung, die von einer Strahlungsquelle kommend über eine Strahlführungseinrichtung 2 in das Handstück 11 eingekoppelt wird, befinden sich ein optisches Element, beispielsweise eine aus Quarzglas bestehende Scheibe 3, die mit einer mikrooptisch wirksamen Fläche 4 versehen ist, sowie eine durch zwei Linsen angedeutet Zoomoptik 5, 6. Die Strahlführungseinrichtung 2 kann sowohl als flexible Lichtleitfaser als auch in Form starrer Übertragungsglieder, die durch Gelenke miteinander verbunden sind, ausgeführt sein.

Der Strahlengang 1 ist beispielhaft zum Zweck der Haarentfernung oder einer anderweitigen kosmetischen Behandlung auf eine Hautstelle 7.1 und damit auf einen Abschnitt einer größeren zu behandelnden Hautfläche 7 gerichtet.

Die Fläche 4 des optischen Elementes 3 sei mit einer refraktiv wirksamen Struktur versehen, die aus einer Vielzahl konkav geformter sphärischer Linsen besteht. Dabei ist die Fläche 4 so in den Strahlengang 1 gestellt, daß der gesamte Strahlengang 1 diese Mikrolinsen passieren muß. Die Linsen haben dabei einen bevorzugten Durchmesser von etwa 0,35 mm und eine bevorzugte Tiefe von 0,005 mm.

Beim Durchgang des Strahlenganges 1 durch die auf der Fläche 4 ausgebildete Mikrolinsenanordnung erfolgt die Aufteilung des beispielsweise von einem Rubinlaser kommenden Laserstrahles in eine Anzahl von Teilstrahlungen, die der Anzahl der Mikrolinsen entspricht. Diese Aufteilung bewirkt, daß der angedeutete kreisrunde Querschnitt 8 des Strahlenganges 1, der eine ungleichmäßige Intensitätsverteilung hat, in eine Strahlung mit gleichmäßiger Intensitätsverteilung innerhalb eines quadratischen Querschnittes 9 transformiert wird (vgl. auch Fig.2). Die Scheibe 3 bewirkt also nicht nur eine Veränderung der Intensitätsverteilung innerhalb des Laserstrahlquerschnittes, sondern zugleich auch eine Änderung der Querschnittsform des Laserstrahles.

Mit der quadratischen Querschnittsform, die der Strahlengang 1 nach dem Passieren der Scheibe 3 aufweist, ist die Strahlung auf die Hautstelle 7.1 gerichtet, wobei mit Hilfe der Zoomoptik 5,6 die Größe der auf der Hautstelle 7.1 auftreffenden Querschnittsfläche 10 beeinflußbar ist. So läßt sich durch Variation der Zoomoptik 5,6 die auf die Hautstelle 7.1 auftreffende Querschnittsfläche 10 vergrößern oder verkleinern. Damit ist auf einfache Weise eine Anpassung an die Fläche der zu behandelnden Hautstelle möglich. Die Größe der Querschnittsfläche 10 beträgt beispielhaft 10mm x 10mm.

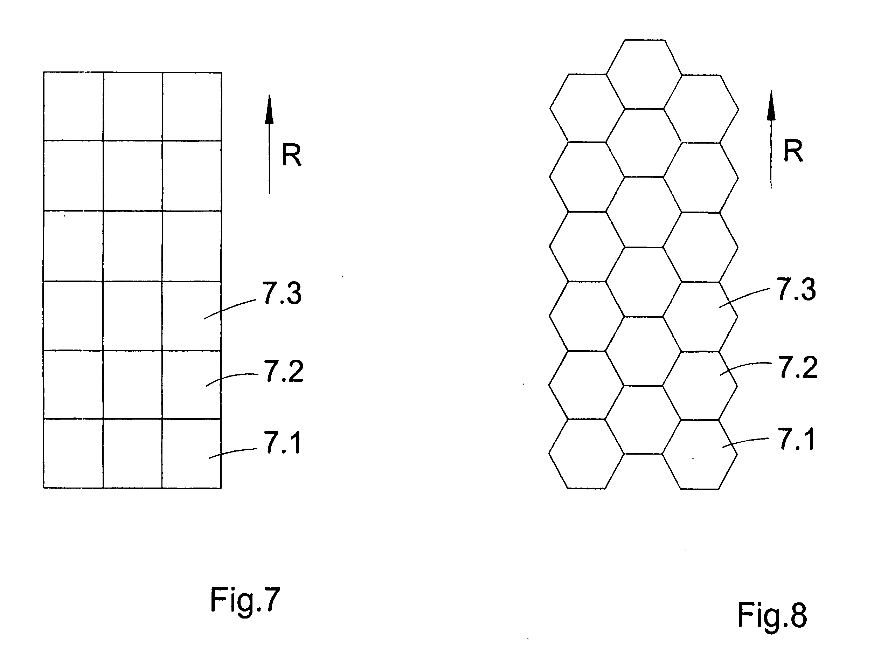

Bei kosmetischen Behandlungen ist in der Regel die insgesamt zu behandelnde Hautfläche größer als die Querschnittsfläche 10, die mit der Zoomoptik 5, 6 einstellbar ist. Das heißt, die gesamte zu behandelnde Hautfläche muß lückenlos durch mehrere aneinandergereihte Querschnittsflächen 10 abgedeckt werden. Das wird erreicht, indem das Handstück nach der Behandlung der Hautstelle 7.1 auf eine benachbarte Hautstelle 7.2 umgesetzt wird, die dann der Laserstrahlung unterworfen ist, danach auf eine nächste Hautstelle 7.3 usw., bis die gesamte Hautfläche, die zur Behandlung vorgesehen war, abgedeckt ist (vgl. Fig.7).

Alternativ zur refraktiv wirksamen Struktur auf der Fläche 4 kann eine diffraktiv wirksame Struktur vorgesehen sein. Damit wird eine Homogenisierung der Intensität innerhalb des Laserstrahlquerschnittes nicht durch Aufteilung des Laserstrahles in eine Vielzahl von Teilstrahlen, sondern durch Phasenänderungen erzielt. Auch dabei läßt sich mit Hilfe der Scheibe 3 beispielsweise ein kreisrunder Strahlquerschnitt mit ungleichmäßiger Intensitätsverteilung in einen quadratischen Querschnitt mit gleichmäßiger Intensitätsverteilung transformieren.

Um nun zu vermeiden, daß insbesondere die empfindlichen Hautschichten durch die Behandlung mit der Laserstrahlung geschädigt werden, ist das Handstück mit einer Einrichtung zum Temperieren der ausgewählten Hautstelle 7.1 vor und/oder nach deren Behandlung ausgestattet. Die Temperierung der Hautstelle 7.1 kann dabei als Erwärmung auf eine vorgegebene Temperatur oder auch als Abkühlung vorgenommen werden. Im weiteren jedoch soll das Ausführungsbeispiel anhand einer Kühlung der Hautstelle 7.1 erläutert werden.

Zu diesem Zweck ist das Handstück 11 mit einer Kontaktfläche 12 versehen. Die Kontaktfläche 12 entspricht in ihrer Form und in ihrer Ausdehnung dem Laserstrahlquerschnitt, wie in Fig.3 zu erkennen ist. Weiterhin ist innerhalb des Handstückes, umschlossen von einem Gehäuse 13, ein Peltierelement 14 vorhanden, dessen kalte Seite 15 mit einem Wärmeleiter 16 in Kontakt steht, an dem die Kontaktfläche 1 2 ausgebildet ist.

Die warme Seite 17 des Peltierelementes 14 ist mit Kanälen 18 versehen, durch die ein Wärmeträgermedium in einem Kreislauf umgepumpt wird. Die Zuführung des Wärmeträgermediums, beispielsweise Wasser, erfolgt über Anschlüsse 19, die mit den Kanälen 18 verbunden sind. Beim Umlaufen des Wärmeträgermediums wird die Wärmeenergie, die beim Betreiben des Peltierelementes 14 von der kalten Seite 15 zur warmen Seite 17 transportiert wird, aus dem Peltierelement 14 nach außen abgeführt. Die zum Betreiben des Peltierelementes 14 erforderliche Energiezufuhr in Form eines elektrischen Potentials ist zeichnerisch nicht dargestellt.

Beim Betreiben der erfindungsgemäßen Anordnung wird zunächst die Kontaktfläche 12 auf eine zur Behandlung ausgewählte Hautstelle 7.1 aufgesetzt. Nach einer vorgegebenen Verweilzeit der Kontaktfläche 12 auf der Hautstelle 7.1, während der der Hautstelle in der beschriebenen Weise Wärme entzogen und die Hautstelle 7.1 somit gekühlt wird, wird die Position des Handstückes 11 nun so verändert, daß sich anstelle der Kontaktfläche 12 die Abstrahlfläche 20, aus welcher die Laserstrahlung aus dem Handstück 11 austritt, über der Hautstelle 7.1 befindet.

Gleichzeitig wird die Kontaktfläche 12 mit einer weiteren zur Behandlung vorgesehenen Hautstelle 7.2 (vgl. Fig.7) in Kontakt gebracht. Während die Kühlung der Hautstelle 7.2 erfolgt, wird über eine Bedieneinheit, die beispielsweise von einem Fußschalter angesteuert werden kann, die Laserquelle in Betrieb genommen, wobei die Behandlung der ersten Hautstelle 7.1 erfolgt.

Nach vorgegebener Einwirkzeit der Laserstrahlung auf die Hautstelle 7.1 wird die Laserquelle wieder abgeschaltet. Nun wird die Position des Handstückes so verändert, daß sich die Abstrahlfläche 20 über der zweiten Hautstelle 7.2 befindet, während die Kontaktfläche 12 mit einer dritten Hautstelle 7.3 in Kontakt gebracht wird. Während der Verweilzeit der Kontaktfläche 12 auf der dritten Hautstelle 7.3 wird wiederum die Laserquelle eingeschaltet und die Behandlung der Hautstelle 7.2, wie bereits beschrieben, vorgenommen.

So wird nacheinander die gesamte zu behandelnde Hautfläche abgetastet, bis deren Behandlung durch die Aneinanderreihung der einzelnen Hautstellen 7.1, 7.2, 7.3 usw. abgeschlossen ist. Dabei wird jeweils eine Hautstelle vorgekühlt, während eine andere mit der Laserstrahlung behandelt wird.

In einer Ausgestaltung der Erfindung kann vorgesehen sein, daß das Handstück 11 mit einer weiteren Kontaktfläche 26 ausgestattet ist, die beispielhaft über einen Wärmeleiter 27 (beides in Fig.1 angedeutet) mit einem weiteren Kühlaggregat, beispielsweise ebenfalls einem Peltierelement, welches in das Handstück 11 eingeordnet ist, in Verbindung steht. Dabei ist es denkbar, die beiden Kontaktflächen 12 und 26 sowie die Querschnittsfläche 10, mit der der Strahlengang 1 auf das Behandlungsareal auftrifft, in einer Geraden anzuordnen, wie das in Fig.3 dargestellt ist.

Wird nun das Handstück 11 während der Behandlung eines größeren Flächenabschnittes in der Richtung R (vgl: Fig.3) von einer Hautstelle zur nächsten versetzt, erfolgt zunächst eine Vorkühlung mit der Kontaktfläche 12, dann nach einem ersten Versetzen des Handstückes 11 die Behandlung der vorgekühlten Hautstelle mit der Laserstrahlung, indem die Laserstrahlung auf diese Hautstelle trifft und beim nachfolgenden zweiten Umsetzen des Handstückes 11, ebenfalls wieder mit einer Schrittweite, die dem Abstand zwischen der Kontaktfläche 12 und der Querschnittsfläche 10 entspricht, eine Nachkühlung der bereits behandelten Hautstelle durch die aufgesetzte Kontaktfläche 26. Die Nachtemperierung dient vor allem der Schonung der Haut.

Die Temperatur der Kontaktfläche 12 und die Intensität der Laserstrahlung sind dabei so aufeinander abgestimmt, daß die Zeit, die für die Vorkühlung erforderlich ist, etwa der Einwirkzeit der Laserstrahlung entspricht, wodurch eine effektive und ohne Stillstandszeiten fortschreitende Behandlung von Hautstelle zu Hautstelle möglich wird.

Die auf der Haut aufsitzenden schmalen Kanten der Filterscheiben dienen dem Operateur als Unterstützung bei der Geradführung des Handstückes von Hautstelle zu Hautstelle und bilden zugleich Schutz vor der schädigenden Laserstrahlung, sofern die Filter auf die Wellenlänge der Strahlung abgestimmt sind.

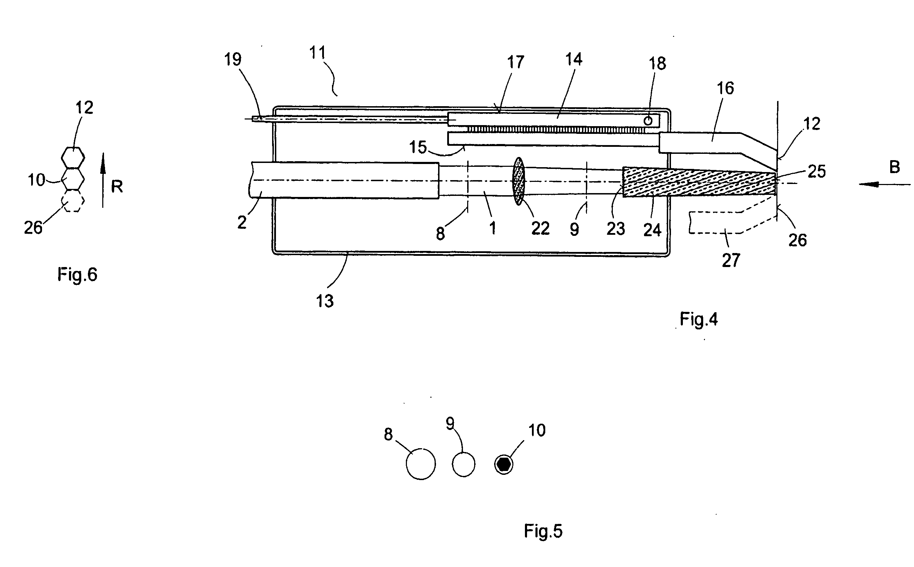

Wie bereits dargestellt, wird mit der Scheibe 3 erreicht, daß nicht nur die Strahlungsintensität bezogen auf den Querschnitt der Laserstrahlung homogenisiert wird, sondern auch eine Querschnittsformung des Strahlenganges 1 vorgenommen wird. Dabei ist es nicht nur (wie bisher beschrieben) möglich, aus einem kreisrunden Querschnitt 8 des Strahlenganges 1 (vgl. Fig.2) einen quadratischen Querschnitt 9 zu formen, sondern es ist auch denkbar, das optische Element 3 so zu gestalten, daß sich an der Position 9 ein sechseckiger Querschnitt des Strahlenganges 1 ergibt, wie das in Fig.5 dargestellt ist.

Eine zweite prinzipielle Ausgestaltungsvariante der erfindungsgemäßen Anordnung zeigt Fig.4. Hier weist der Strahlengang 1, ebenfalls über eine Strahlführungseinrichtung 2 eingekoppelt, zunächst auch wieder einen kreisrunden Querschnitt 8 mit inhomogener Verteilung der Strahlungsintensität auf. Jedoch ist abweichend zur Ausgestaltungsvariante nach Fig.1 in den Strahlengang 1 eine Sammellinse 22 gestellt, durch welche der Laserstrahl fokussiert auf die Einstrahlfläche 23 eines strahlführenden Stabes 24 gerichtet ist.

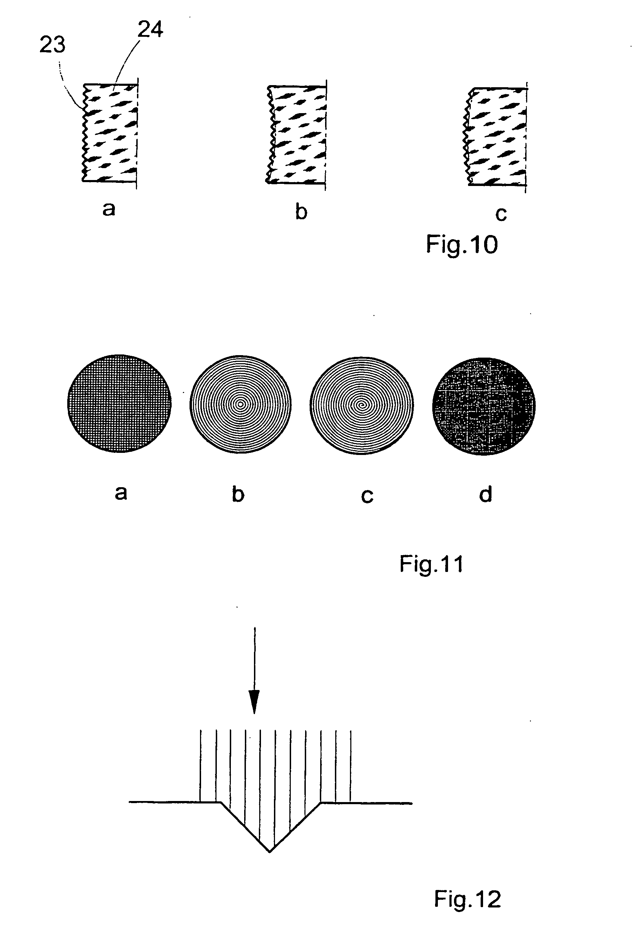

Die Einstrahlfläche 23 ist mit einer Struktur aus nebeneinander angeordneten Mikrolinsen versehen (vgl. auch Fig.10 bis Fig.12). Dabei wird die Laserstrahlung auch hier beim Durchgang durch die Einstrahlfläche 23 in eine der Vielzahl von Mikrolinsen entsprechende Anzahl Teilstrahlungen aufgeteilt und damit eine Homogenisierung der Intensitätsverteilung bewirkt. Innerhalb des strahlführenden Stabes 24 wird die Laserstrahlung durch Totalreflexion weitergeleitet, wobei eine weitere Homogenisierung erzielt wird. An der Abstrahlfläche 25, die während der Behandlung nahe der ausgewählten Hautstelle oder im unmittelbaren Kontakt mit dieser positioniert ist, ist ein Laserstrahl verfügbar, dessen Querschnitt bis in die Randbereiche hinein eine gleichmäßige Strahlungsintensität aufweist. Damit ist eine gleichmäßige Behandlung der mit dieser Laserstrahlung beaufschlagten Hautstelle gewährleistet.

Die Strahlformung erfolgt hier innerhalb des Stabes 24, indem aus dem vor Eintritt in die Einstrahifläche 23 noch kreisrunden Querschnitt 9 des Strahles durch entsprechende Gestaltung des Stabquerschnittes eine Veränderung des Strahlquerschnittes in eine vieleckige Querschnittsform (Querschnitt 10) der Abstrahlfläche 25 erzielt wird. Dabei ist die Abstrahlfläche 25 gegenüber der Einstrahlfläche 23 reduziert, wobei der kreisrunde Querschnitt über wenigstens 90% der Länge des Stabes erhalten bleiben und erst auf dem verbleibenden Längenabschnitt eine Reduzierung und/oder Formänderung des Querschnitts vorgesehen sein sollte.

Auch in dieser Ausgestaltungsvariante ist das Handstück 11 wiederum mit einer Kontaktfläche 12 versehen, die über einen Wärmeleiter 16 mit der kalten Seite 15 eines Peltierelementes 14 gekoppelt ist. Wie bereits anhand Fig.1 beschrieben, wird auch hier die Wärmeenergie von der warmen Seite 17 mit Hilfe eines durch Kanäle 18 umlaufenden Wärmeträgermediums abgeführt.

Wie in der anhand Fig.1 beschriebenen Ausgestaltung kann auch hier vorgesehen sein, daß (wie zeichnerisch mit unterbrochenen Linien angedeutet) eine weitere Kontaktfläche 26, ein weiterer Wärmeleiter 27, ein weiteres Kühlaggregat usw. vorhanden sind. Mit einem Handstück dieser hier beschriebenen Ausgestaltung kann ebenso verfahren werden wie mit dem Handstück nach der Ausgestaltungsvariante gemäß Fig.1.

Wie in Fig.6 dargestellt ist hier beispielhaft vorgesehen, daß der Querschnitt der Abstrahlfläche 25 und der Querschnitt der Kontaktfläche 12 (wie auch die Kontaktfläche 26) sechseckig ausgeführt sind. Damit ist, ähnlich wie in der vorbeschriebenen Weise, eine Aneinanderreihung der nacheinander behandelten Hautstellen möglich, bis eine gesamte zu behandelnde Hautfläche lückenlos abgedeckt ist (vgl. Fig.8).

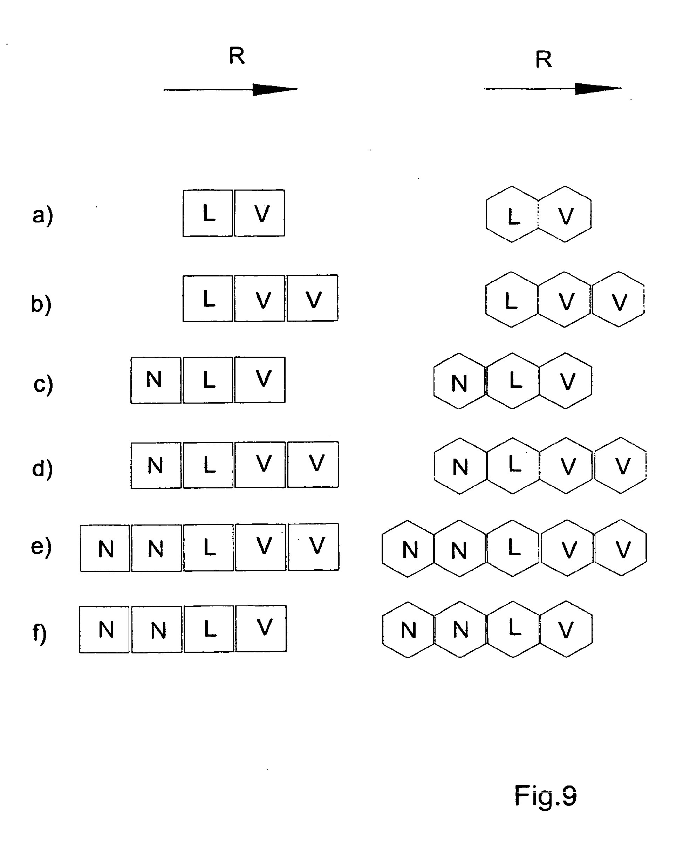

Die Fig.9 zeigt verschiedenartige Gestaltungsmöglichkeiten im Hinblick auf die Nachbarschaft vortemperierter, behandelter und nachtemperierter Hautstellen. Hierin bedeuten jeweils L = Lasern im Augenblick der Behandlung, V = Vortemperieren zur Vorbereitung des Laserns und N = Nachtemperieren zur Nachbehandlung der bereits durch Laserstrahlung beeinflußten Hautstelle. Die einzelnen Flächenabschnitte können unterschiedliche geometrische Formen und auch voneinander abweichende Flächenausdehnung haben. Der Übersichtlichkeit halber sind hier allerdings nur quadratische und sechseckige Formen etwa gleicher Ausdehnung dargestellt.

Die Einstrahlfläche 23 kann, wie beispielhaft in Fig.10 dargestellt, plan (Fig.10a), konkav (Fig.10b) oder auch konvex (Fig.10c) geformt sein. Damit ist in Zusammenwirkung mit der Auswahl der Struktur, die auf die Einstrahlfläche 23 aufgebracht ist, eine gezielte Beeinflussung der Strahlungsintensität wie auch des Strahlquerschnittes möglich.

Fig.11 zeigt mehrere Variationen der Einstrahlflächen 4,23 in Draufsicht. Hier sind, ebenfalls beispielhaft, verschiedene mikrooptisch wirksame Strukturen dargestellt, die nicht maßstäblich und auch zum Zweck der Verdeutlichung wesentlich vergrößert gezeichnet sind. So zeigt Fig.11a die Anordnung einer Vielzahl von linsenartigen Vertiefungen, die statistisch über die gesamte Einstrahlfläche 23 verteilt sind.

In Fig.11b besteht die Struktur aus zentrisch angeordneten Rillen, die jeweils einen keilförmigen Querschnitt aufweisen. Ein solcher Querschnitt ist beispielhaft in Fig.12 dargestellt. In Fig.11c ist eine aus einer spiralförmigen Rille bestehende Mikrostruktur vorgesehen. Fig.11d dagegen zeigt ein Netz aus sich kreuzenden, geradlinigen Rillen, die ebenfalls den Querschnitt gemäß Fig.12 aufweisen können.

## Patentansprüche

1. Dermatologisches Handstück, von dem ein Laserstrahl auf die Oberfläche einer ausgewählten Hautfläche gerichtet ist, wobei die Haut der Einwirkung des Laserstrahles unterzogen wird und wobei der Laserstrahl in zeitlicher Folge fortschreitend auf einzelne, dem Laserstrahlquerschnitt entsprechende, in ihrer Gesamtheit die ausgewählte Hautfläche überdeckende Hautstellen (7.1,7.2,7.3) gerichtet wird ist, ausgestattet mit einer Einrichtung zum Temperieren der Hautfläche, bei der
- mindestens eine temperierte Kontaktfläche (12) seitlich neben dem auf eine Hautstelle (7.2) gerichteten Laserstrahl positioniert ist, wobei
- die Form der Kontaktfläche (12) der Form des Laserstrahlquerschnittes entspricht,
- *die flächige Ausdehnung der Kontaktfläche (12) dem 0,5- bis 2-fachen der flächigen Ausdehnung der Querschnittsfläche (10) des Laserstrahles entspricht,* und
- der Abstand zwischen Laserstrahl und Kontaktfläche (12) so bemessen ist, daß in zeitlich aufeinander folgenden Schritten der Laserstrahl und die neben dem Laserstrahl positionierte Kontaktfläche (12) so auf die Hautstellen (7.1,7.2,7.3) gerichtet sind, daß
- jeweils während einer vorgegebenen Einwirkzeit
- der Laserstrahl ortsunveränderlich auf eine Hautstelle (7.2) gerichtet ist und dabei zugleich die Kontaktfläche (12)
- mit einer zuvor der Laserstrahlung ausgesetzten Hautstelle (7.1) oder
- mit einer nachfolgend der Laserstrahlung auszusetzende Hautstelle (7.3) in Berührung steht.

2. Dermatologisches Handstück nach einem der vorgenannten Ansprüche, **dadurch gekennzeichnet, daß** mindestens eine gekühlte Kontaktfläche (12) vorhanden ist.

3. Dermatologisches Handstück nach einem der vorgenannten Ansprüche, **dadurch gekennzeichnet, daß** die Ausdehnung der Kontaktfläche (12) um den Faktor 1,2 größer ist als die Ausdehnung der Querschnittsfläche (10) des Laserstrahles.

4. Dermatologisches Handstück nach einem der vorgenannten Ansprüche, **dadurch gekennzeichnet, daß** die Ausdehnung der Kontaktfläche (12) um den Faktor 0,7 kleiner ist als die Ausdehnung der Querschnittsfläche (1 0) des Laserstrahles.

5. Dermatologisches Handstück nach einem der vorgenannten Ansprüche, **dadurch gekennzeichnet, daß** die Einrichtung zum Temperieren über ein Kühl- und/oder Heizaggregat verfügt, das mit der Kontaktfläche (12) wärmeleitend, vorzugsweise über ein in einem Kreislauf umgewälztes Wärmeträgermedium, in Verbindung steht.

6. Dermatologisches Handstück nach Anspruch 5, **dadurch gekennzeichnet, daß** als Kühlaggregat ein Peltierelement (14) vorgesehen ist, dessen kalte Seite (15) wärmeleitend mit der Kontaktfläche (12) und dessen warme Seite (17) mit einem Kühlkreislauf in Verbindung steht.

7. Dermatologisches Handstück nach Anspruch 5, **dadurch gekennzeichnet, daß** die Kontaktfläche (12) wärmeleitend mit einem sich entspannenden und dabei abkühlenden Gas, vorzugsweise N₂ in Verbindung steht.

8. Dermatologisches Handstück nach einem der vorgenannten Ansprüche, **dadurch gekennzeichnet, daß** ein Temperatursensor zur Erfassung der Temperatur der Kontaktfläche (12) und/oder der ausgewählten Hautstelle (7.1,7.2,7.3) vorhanden ist, wobei vorzugsweise der Signalausgang des Temperatursensors mit einem Schwellwertschalter verbunden ist, durch den ein Einschaltsignal an das Kühl- und/oder Heizaggregat ausgegeben wird, sobald ein voreingestellter Temperaturwert unter- bzw. überschritten ist.

9. Dermatologisches Handstück nach Anspruch 8, **dadurch gekennzeichnet, daß** das Einschaltsignal des Schwellwertschalters außerdem an einem Signalgeber anliegt, durch den ein sinnlich wahrnehmbares, vorzugsweise akustisches Signal ausgegeben wird, sobald ein voreingestellter Temperaturwert unter- bzw. überschritten ist.

10. Dermatologisches Handstück nach einem der vorgenannten Ansprüche, **dadurch gekennzeichnet, daß** mindestens zwei Kontaktflächen (12, 26) vorgesehen sind, deren Zentren mit dem Zentrum der Querschnittsfläche (10) auf einer Geraden liegen, wobei der Abstand zwischen den Zentren der Querschnittsfläche (10) und einer der Kontaktflächen (12, 26) etwa dem Abstand zwischen zwei unmittelbar benachbarten Hautstellen (7.1, 7.2) entspricht.

11. Dermatologisches Handstück nach Anspruch 10, **dadurch gekennzeichnet, daß** der Laserstrahl zwischen den beiden Kontaktflächen (12, 26) austritt.

12. Dermatologisches Handstück nach einem der vorgenannten Ansprüche, **dadurch gekennzeichnet, daß** Mittel zur Veränderung der Intensitätsverteilung innerhalb der Querschnittsfläche (10) und/oder mit Mittel zur Veränderung der Querschnittsfläche (10) hinsichtlich Form und/oder Ausdehnung vorhanden sind.

13. Dermatologisches Handstück nach Anspruch 12, **dadurch gekennzeichnet, daß** als Mittel mindestens ein optisches Element mit einer im Mikrometerbereich strukturierten und **dadurch** mikrooptisch diffraktiv und/oder refraktiv wirksamen Einstrahlfläche (4,23) vorgesehen ist.

14. Dermatologisches Handstück nach Anspruch 13, **dadurch gekennzeichnet, daß** die Einstrahlfläche (4,23) eine diffraktiv wirksame Struktur aufweist, bei der die Strukturbreite etwa der Wellenlänge der zur Behandlung genutzten Laserstrahlung entspricht und die
- als variierendes Höhenprofil mit streifenförmigen, kreuzförmigen, trichterförmigen und/oder anderweitig geformten Erhebungen,
- als variierter Brechungsindex und/oder
- in Form eines variierten Absorptionskoeffizienten ausgebildet ist.

15. Dermatologisches Handstück nach Anspruch 13, **dadurch gekennzeichnet, daß** die Einstrahlfläche (4,23) eine refraktiv wirksame Struktur aus sphärischen, asphärischen, zylindrischen und/oder elliptischen, hexagonal und/oder orthogonal angeordneten, konkav und/oder konvex geformten Linsen aufweist.

16. Dermatologisches Handstück nach einem der Ansprüche 13 bis 15, **dadurch gekennzeichnet, daß** dem optischen Element eine Einrichtung zur Strahlfokussierung, bevorzugt eine Sammellinse (22), vor- oder nachgeordnet ist.

17. Dermatologisches Handstück nach einem der Ansprüche 13 bis 16, **dadurch gekennzeichnet, daß** das optische Element als strahlführender Stab (24) ausgebildet ist, der über eine refraktiv strukturierte Einstrahlfläche (23) verfügt, in dem die Strahlung durch Totalreflexion weitergeleitet und aus einer Abstrahlfläche (25) auf die Hautfläche abgestrahlt wird.

18. Dermatologisches Handstück nach Anspruch 17, **dadurch gekennzeichnet, daß** die Einstrahlfläche (23) gewölbt, bevorzugt konkav, besonders bevorzugt konvex ausgebildet ist.

19. Dermatologisches Handstück nach Anspruch 17 oder 18, **dadurch gekennzeichnet, daß** die Abstrahlfläche (25) einen kreisrunden Querschnitt aufweist.

20. Dermatologisches Handstück nach Anspruch 17 oder 18, **dadurch gekennzeichnet, daß** die Abstrahlfläche (25) einen vieleckigen Querschnitt, bevorzugt einen quadratischen, besonders bevorzugt einen sechseckigen Querschnitt aufweist.

21. Dermatologisches Handstück nach einem der vorgenannten Ansprüche, **dadurch gekennzeichnet, daß** zwischen der Abstrahlfläche (25) und der Hautfläche eine Schicht aus einem für die Laserstrahlung transparenten Medium, bevorzugt ein Gel, besonders bevorzugt ein Ultraschallgel, vorgesehen ist.

22. Dermatologisches Handstück nach Anspruch 21, **dadurch gekennzeichnet, daß** ein Ultraschallgel bis zu maximal einer Dicke von 1 mm auf die Hautfläche aufgetragen wird.

## Claims

1. Dermatological hand piece by which a laser beam is directed onto the surface of a selected skin area, wherein the skin is subjected to the action of the laser beam and wherein the laser beam is directed successively onto individual skin sites (7.1, 7.2, 7.3) corresponding to the laser beam cross-section and covering the selected skin area in its entirety, equipped with a device for moderating the temperature of the skin area, in which
- at least one temperature-moderated contact surface (12) is positioned laterally adjacent to the laser beam which is directed onto a skin site (7.2), wherein
- the shape of the contact surface (12) corresponds to the shape of the laser beam cross-section,
- the surface dimensions of the contact surface (12) correspond to 0.5 to 2 times the surface dimensions of the cross-sectional surface (10) of the laser beam, and
- the distance between the laser beam and contact surface (12) is dimensioned in such a way that in successive steps the laser beam and the contact surface (12) positioned adjacent to the laser beam are directed onto the skin sites (7.1, 7.2, 7.3) in such a way that
- in each case during a predetermined action time
- the laser beam is directed without change of location onto a skin site (7.2) and thereby at the same time the contact surface (12) is in contact with
- with a skin site (7.1) previously exposed to the laser radiation or
- with a skin site (7.3) to be subjected subsequently to the laser radiation.

2. Dermatological hand piece as claimed in any one of the preceding claims, **characterised in that** there is at least one cooled contact surface (12).

3. Dermatological hand piece as claimed in any one of the preceding claims, **characterised in that** the dimensions of the contact surface (12) are greater than the dimensions of the cross-sectional area (10) of the laser beam by a factor of 1.2.

4. Dermatological hand piece as claimed in any one of the preceding claims, **characterised in that** the dimensions of the contact surface (12) are smaller than the dimensions of the cross-sectional area (10) of the laser beam by a factor of 0.7.

5. Dermatological hand piece as claimed in any one of the preceding claims, **characterised in that** a device for temperature moderation has a cooling unit and/or heating unit which communicates in a heat-conducting manner with the contact surface (12), preferably by way of a heat carrying medium circulating in a circuit.

6. Dermatological hand piece as claimed in Claim 5, **characterised in that** a Peltier element (14) is provided as cooling unit, wherein the cold side (15) thereof communicates in a heat-conducting manner with the contact surface (12) and the warm side (17) communicates with a cooling circuit.

7. Dermatological hand piece as claimed in Claim 5, **characterised in that** the contact surface (12) communicates in a heat-conducting manner with an expanding and thereby cooling gas, preferably N₂.

8. Dermatological hand piece as claimed in any one of the preceding claims, **characterised in that** a temperature sensor is provided for detection of the temperature of the contact surface (12) and/or of the selected skin site (7.1, 7.2, 7.3), wherein the signal output of the temperature sensor is preferably connected to a threshold switch, by which a switch-on signal is sent to the cooling and/or heating unit as soon as the temperature falls below or exceeds a preset temperature value.

9. Dermatological hand piece as claimed in Claim 8, **characterised in that** the switch-on signal of the threshold switch is also applied to a signal transmitter by which a perceptible, preferably acoustic signal is emitted as soon as the temperature falls below or exceeds a preset temperature value.

10. Dermatological hand piece as claimed in any one of the preceding claims, **characterised in that** at least two contact surfaces (12, 26) are provided, the centres of which lie on a straight line with the centre of the cross-sectional surface (10), wherein the distance between the centres of the cross-sectional surface (10) and one of the contact surfaces (12, 26) corresponds approximately to the distance between two directly adjacent skin sites (7.1, 7.2).

11. Dermatological hand piece as claimed in Claim 10, **characterised in that** the laser beam exits between the two contact surfaces (12, 26).

12. Dermatological hand piece as claimed in any one of the preceding claims, **characterised in that** means are provided for changing the intensity distribution within the cross-sectional area (10) and/or means are provided for changing the cross-sectional area (10) with regard to shape and/or dimensions.

13. Dermatological hand piece as claimed in Claim 12, **characterised in that** the means are provided in the form of at least one optical element with an irradiation surface (4, 23) which is structured in the micrometer range and as a result is micro-optically diffractive and/or refractive.

14. Dermatological hand piece as claimed in Claim 13, **characterised in that** the irradiation surface (4, 23) has a diffractively active structure in which the structure width corresponds approximately to the wavelength of the laser radiation used for treatment and which is constructed
- as a varying height profile with raised areas which strip-shaped, cross-shaped, funnel-shaped and/or of other shapes,
- as a varying index of refraction and/or
- in the form of a varying absorption coefficient.

15. Dermatological hand piece as claimed in Claim 13, **characterised in that** the irradiation surface (4, 23) has a refractively active structure formed from lenses of concave and/or convex shape which are spherical, aspherical, cylindrical and/or elliptical and are disposed hexagonally and/or orthogonally.

16. Dermatological hand piece as claimed in any one of Claims 13 to 15, **characterised in that** a device for beam focusing, preferably a convergent lens (22), is disposed in front of or behind the optical element.

17. Dermatological hand piece as claimed in any one of Claims 13 to 16, **characterised in that** the optical element is constructed as a beam-guiding rod (24) which has a refractively structured irradiation surface (23) in which the beam is sent by total reflection and is radiated onto the skin surface from an emission surface (25).

18. Dermatological hand piece as claimed in Claim 17, **characterised in that** the irradiation surface (23) is curved, preferably concave, particularly preferably convex.

19. Dermatological hand piece as claimed in Claim 17 or 18, **characterised in that** the emission surface (25) has a circular cross-section.

20. Dermatological hand piece as claimed in Claim 17 or 18, **characterised in that** the emission surface (25) has a polygonal cross-section, preferably a square cross-section, particularly preferably a hexagonal cross-section.

21. Dermatological hand piece as claimed in any one of the preceding claims, **characterised in that** a layer of a medium which is transparent to laser radiation, preferably a gel, particularly preferably an ultrasound gel, is provided between the emission surface (25) and the skin surface.

22. Dermatological hand piece as claimed in Claim 21, **characterised in that** an ultrasound gel is applied to the skin surface up to a maximum thickness of 1 mm.

## Revendications

1. Pièce à main dermatologique, de laquelle un faisceau laser est dirigé sur la superficie d'une surface de peau choisie, sachant que la peau est soumise à l'effet du faisceau laser et sachant que le faisceau laser est dirigé en séquence chronologique en progression sur des emplacements individuels de la peau (7.1, 7.2, 7.3) correspondant à la section du faisceau laser, qui recouvrent dans leur ensemble la surface de peau choisie, équipée d'un dispositif pour l'équilibrage de température de la surface de peau, dans laquelle
au moins une surface de contact (12) équilibrée en température est positionnée latéralement à côté du faisceau laser dirigé sur un emplacement de la peau (7.2), sachant que
la forme de la surface de contact (12) correspond à la forme de la section du laser,
l'extension superficielle de la surface de contact (12) correspond à 0,5 à 2 fois l'extension superficielle de la surface de section (10) du faisceau laser, et
la distance entre le faisceau laser et la surface de contact (12) est dimensionnée de telle sorte que, dans des étapes se suivant chronologiquement l'une l'autre, le faisceau laser et la surface de contact (12) positionnée à côté du faisceau laser sont dirigés sur les emplacements de la peau (7.1, 7.2, 7.3) de telle sorte que
pendant une durée d'action chaque fois prédéfinie
le faisceau laser est dirigé avec une localisation invariable sur un emplacement de la peau (7.2) et ce faisant, en même temps, la surface de contact (12) se trouve en contact
avec un emplacement de la peau (7.1) précédemment exposé au rayonnement laser ou
avec un emplacement de la peau (7.3) à exposer ultérieurement au rayonnement laser.

2. Pièce à main dermatologique selon la revendication 1, **caractérisée en ce qu'**il existe au moins une surface de contact (12) refroidie.

3. Pièce à main dermatologique selon l'une des revendications précédentes, **caractérisée en ce que** l'extension de la surface de contact (12) est plus grande d'un facteur 1,2 que l'extension de la surface de section (10) du faisceau laser.

4. Pièce à main dermatologique selon l'une des revendications précédentes, **caractérisée en ce que** l'extension de la surface de contact (12) est plus petite d'un facteur 0,7 que l'extension de la surface de section (10) du faisceau laser.

5. Pièce à main dermatologique selon l'une des revendications précédentes, **caractérisée en ce que** le dispositif pour l'équilibrage des températures dispose d'une unité de refroidissement et/ ou de chauffage qui se trouve en liaison avec la surface de contact (12) de façon conductrice de la chaleur, de préférence par l'intermédiaire d'un fluide caloporteur pompé en circuit fermé.

6. Pièce à main dermatologique selon la revendication 5, **caractérisée en ce qu'**un élément de Peltier (14), dont le côté froid (15) se trouve en liaison avec la surface de contact (12) de façon conductrice de la chaleur, et dont le côté chaud (17) se trouve en contact avec un circuit de refroidissement, est prévu comme unité de réfrigération.

7. Pièce à main dermatologique selon la revendication 5, **caractérisée en ce que** la surface de contact (12) se trouve en liaison de façon conductrice de la chaleur avec un gaz se détendant et se refroidissant en même temps, de préférence N₂.

8. Pièce à main dermatologique selon l'une des revendications précédentes, **caractérisée en ce qu'**il existe un capteur de température pour la saisie de la température de la surface de contact (12) et/ou des emplacements choisis de la peau (7.1, 7.2, 7.3), sachant que, de préférence, la sortie de signal du capteur de température est reliée à un interrupteur à valeur de seuil par lequel un signal de mise du contact est émis à l'unité de refroidissement et/ou de chauffage dès qu'une certaine valeur de température présélectionnée est dépassée par valeur inférieure, ou supérieure.

9. Pièce à main dermatologique selon la revendication 8, **caractérisée en ce que** le signal de mise du contact de l'interrupteur à valeur de seuil est présent en outre sur un générateur de signal par lequel un signal percevable par les sens, de préférence un signal sonore, est émis dès qu'une certaine valeur de température présélectionnée est dépassée par valeur inférieure, respectivement supérieure.

10. Pièce à main dermatologique selon l'une des revendications précédentes, **caractérisée en ce qu'**au moins deux surfaces de contact (12, 26) sont prévues, dont les centres se trouvent sur une droite avec le centre de la surface de section (10), sachant que la distance entre les centres de la surface de section (10) et une des surfaces de contact (12, 26) correspond approximativement à la distance entre deux emplacements de la peau (7.1, 7.2) immédiatement voisins.

11. Pièce à main dermatologique selon la revendication 10, **caractérisée en ce que** le faisceau laser sort entre les deux surfaces de contact (12, 26).

12. Pièce à main dermatologique selon l'une des revendications précédentes, **caractérisée en ce qu'**il existe des moyens de modification de la distribution des intensités à l'intérieur de la surface de section (10) et/ou des moyens de modification de la surface de section (10) du point de vue de la forme et/ou de l'extension.

13. Pièce à main dermatologique selon la revendication 12, **caractérisée en ce que**, comme moyen, il est prévu au moins un élément optique avec une surface d'incidence du rayonnement (4, 23) structurée de l'ordre de grandeur du micromètre et par là à effet micro-optiquement diffractant et/ou réfractant.

14. Pièce à main dermatologique selon la revendication 13, **caractérisée en ce que** la surface d'incidence du rayonnement (4, 23) présente une structure à effet diffractant, pour laquelle la largeur de structure correspond à peu près à la longueur d'onde du rayonnement laser utilisé pour le traitement et qui est configurée
comme profil de hauteur variable avec des saillies en forme de bandes, en forme de croix, en forme d'entonnoir et/ ou d'une autre forme,
comme indice de réfraction variable et/ou
de la forme d'un coefficient d'absorption variable.

15. Pièce à main dermatologique selon la revendication 13, **caractérisée en ce que** la surface d'incidence du rayonnement (4, 23) présente une structure à effet de réfraction en lentilles sphériques, asphériques, cylindriques et/ou elliptiques, disposées de façon hexagonale et/ou orthogonale, de forme concave et/ ou convexes.

16. Pièce à main dermatologique selon l'une des revendications 13 à 15, **caractérisée en ce qu'**à l'élément optique est associé en amont ou en aval un dispositif pour la focalisation du faisceau, de préférence une lentille collectrice (22).

17. Pièce à main dermatologique selon l'une des revendications 13 à 16, **caractérisée en ce que** l'élément optique est configuré comme barreau (24) conducteur du faisceau, qui dispose d'une surface d'incidence du faisceau (23) structurée pour réfraction, dans lequel le rayonnement est réacheminé par réflexion totale et est rayonné sur la peau depuis une surface de sortie du faisceau (25).

18. Pièce à main dermatologique selon la revendication 17, **caractérisée en ce que** la surface d'incidence du faisceau (23) est configurée courbe, de préférence concave, de façon particulièrement préférée convexe.

19. Pièce à main dermatologique selon la revendication 17 ou 18, **caractérisée en ce que** la surface de sortie du faisceau (25) présente une section circulaire.

20. Pièce à main dermatologique selon la revendication 17 ou 18, **caractérisée en ce que** la surface de sortie du faisceau (25) présente une section polygonale, de préférence une section carrée, de façon particulièrement préférée une section hexagonale.

21. Pièce à main dermatologique selon l'une des revendications précédentes, **caractérisée en ce qu'**entre la surface de sortie du faisceau (25) et la surface de la peau est prévue une couche en un milieu transparent pour le rayonnement laser, de préférence un gel, de façon particulièrement préférée un gel pour ultrasonographie.

22. Pièce à main dermatologique selon la revendication 21, **caractérisée en ce qu'**un gel pour ultrasonographie est appliqué sur la surface de la peau jusqu'à une épaisseur de 1 mm au maximum.
